# EUROPEAN PATENT SPECIFICATION

(11) **EP 2 404 620 B1**
(45) Date of publication and mention of the grant of the patent: **14.12.2016**
(21) Application number: 11172507.3
(22) Date of filing: 04.07.2011
(51) Int. Cl.: A61L 2/10, A61G 12/00, A61L 2/26

(54) **Storage device**
Lagerbehälter
Récipient de stockage

(30) Priority: 05.07.2010 IT MI20101230
(43) Date of publication of application: 11.01.2012
(73) Proprietor: Lucini Surgical Concept S.r.L., 20129 Milano (IT)
(72) Inventor: Lucini, Flavio, 20021 BOLLATE (Milan) (IT)
(74) Representative: Lunati & Mazzoni S.r.L.

(56) References cited:
- EP-A1- 1 905 343
- WO-A1-2010/027527
- US-A- 4 583 795
- US-A1- 2002 096 845

## Description

The present invention relates to a storage device of the type pointed out in the preamble of the first claim.

In particular, the invention relates to a trolley or a piece of furniture adapted to receive, at the inside thereof, medical devices such as containers for sanitary purposes, ISO forms, catheters, apparatus in general, scalpels, needles and forceps, capable of ensuring a high hygiene degree.

Therefore it can be used inside medical structures, such as laboratories, hospitals or pharmaceutical or chemical industries in order to handle and/or preserve medical equipment.

As known, presently one of the most important problems concerning health is represented by hospital infections. These infections are a rather heterogeneous assembly of conditions different from each other from a microbiologic and epidemiological point of view, and have a high impact on health and sanitary costs. In addition they are an indication of the quality of the service offered to patients.

Hospital infections, caused by opportunistic micro-organism present in the environment which usually do not give origin to infections, can arise on patients during admission and hospitalisation or, in some cases, after the patient's discharge and can be more or less serious, sometimes even lethal. Hospital infections can also concern health workers acting in contact with patients.

Therefore it is necessary that appropriate measures be adopted not only for treating the hospitalised persons but also for preventing diffusion of hospital infections between the staff supplying assistance and cares.

Studies carried out prove that through an appropriate control on the medical devices used, it is possible to prevent at least 30% of the arising hospital infections, which will bring about a reduction in the cost and improvement of the health service. In fact, since hospital infections adversely affect medical costs and extend the patients' hospitalisation, they ultimately have an important influence on the capability of the hospital institutions to ensure admission to other patients.

There are plenty of micro-organism sources that can give rise to infections: the structures themselves, ventilation and aeration systems, water flows, treatment of tissues and laboratory samples, personal and environmental hygiene, surgical operations and sanitary instruments such as scalpels, bandages, catheters and valves.

A fundamental role for the above mentioned infection sources is played by the sterility of the medical devices that, being adapted to come into direct contact with the internal organs, constitute the most critical micro-organism vehicle. For this reason the availability of sterile medical devices under any condition and at any moment is a fundamental necessity.

Presently, medical device before carrying out invasive procedures or at all events before being introduced into the patient's environment, are packed into specific envelopes or containers and then sterilised following specific technologies submitted to severe regulations and procedures. At the end of this operation the devices are directly used or stored in suitable fixed or movable structures, such as shelves, cabinets, trolleys, waiting for their cycle of use.

These structures can be of two types: open structures such as shelves, which expose the products to the external environment and are used as holding structures and for organisation; closed structures that, in addition to the aforesaid functions, must protect the medical devices against impacts or envelope tearing and, above all, against noxious environmental agents such as dust, bacteria, polluting agents, etc.

It is therefore of the fundamental importance that not only scalpels and the other medical devices be sterile, but that also the different support shelves be hygienically irreproachable, i.e. adapted to be easily sanitised and having a low bacterium-retention potential, so as to prevent bacteria and micro-organisms from moving from the shelves/supports to the medical devices and then to the patient thus infecting him/her.

Trolleys/shelves/cabinets should be frequently sanitised manually or through automatic washing tunnels.

A further fundamental aspect enabling availability of a hygienically irreproachable holding device is represented by the material used for manufacturing it .

Presently, among the materials used it is possible to distinguish: stainless steel that ensures good washability and sterilisation and is in addition a material susceptible of self-passivation; aluminium that is light and easily washable and oxidisable and can be painted; iron that is cheap and can be painted; and polymeric materials that can be worked easily and have low manufacturing costs. Some examples of known storage devices are described in US 2002096845, US 4583795 A, WO 2010027527 A1, WO 2010027527 A1 and EP 1905343. The known art mentioned above has some important drawbacks.

In fact, the materials presently used have many faults. In particular, stainless steel due to the forming folds of the sheets, has hidden spaces where bacteria and dust lay down and has a high weight; aluminium, in addition to the steel faults, is not susceptible of self-passivation and painting and/or oxidation of same has a tendency to deteriorate; iron, in addition to the above defects, is subjected to get rusty or, if painted, easily exfoliates; and polymeric material has high costs and deteriorates easily in the washing tunnels.

Another defect of the above mentioned systems consists in that washing both automatic and manual is often made inefficient by the construction technology of said systems or the materials used, and the washability feature in thermodisinfectors can be only and exclusively found in products specifically made and not in generic products.

Another important defect of known devices is represented by the reduced adaptability to the different uses, due to a substantially unmodifiable structure. A further problem belonging to storage devices resides in the construction geometries, the materials used and the surface treatments of same that restrict the attitude to hygiene of the devices themselves and the attitude to efficient washing and thermodisinfection.

In addition, known devices can be hardly adapted to the different use conditions and therefore a medical structure is obliged to purchase a high number of devices.

Under this situation, the technical task underlying the present invention is to conceive a storage and transport device capable of substantially obviating the mentioned drawbacks.

Within the scope of this technical task it is an important aim of the invention to conceive a sturdy storage device of low cost that can be easily adapted to every use and is able to ensure a high hygiene level.

Another important aim of the invention is to conceive a storage device characterised by high sanitisation capability, reduced bacterium retention and high attitude to holding and transportation of medical devices suitably packed. In other words, it is a fundamental objective of the invention to provide a device both ensuring a high hygiene degree at the end of the cleaning operation and maintaining this aseptic character for a long time.

A further aim of the invention is to accomplish a storage device of easy cleaning, i.e. not requiring complicated washing operations and therefore adapted to be washed both in the washing tunnel and manually.

Another object of the invention is to limit use of the sterilisation machines thereby reducing the maintenance costs for same.

A further objective is to create a structure that, while maintaining the conditions necessary to achieve sanitisation, is adapted to be used with different operating measures, in a modular manner and with possibility of different configurations.

The technical task mentioned and the aims specified are achieved by a storage device as claimed in the appended claim 1.

Preferred embodiments are highlighted in the sub-claims.

The features and advantages of the invention are hereinafter clarified by the detailed description of preferred embodiments of the invention, with reference to the accompanying drawings, in which:
**Fig. 1** shows a view of the storage device in accordance with the invention;
**Fig. 2** is a detail seen in section of the device;
**Fig. 3** is an exploded view of the storage device in accordance with the invention;
**Fig. 4** highlights an exploded view of a further storage device; and
**Fig. 5** shows a detail of the storage device according to the invention.

With reference to the drawings, the storage device according to the invention is generally identified by reference numeral **1**.

In particular, the storage device 1 defines at least one rest surface **1a** on which items of any shape and size can be easily disposed and picked up. In particular this rest surface 1a is adapted to receive medical devices such as scalpels, needles, forceps and defibrillators or, alternatively, trays with drinks or food.

The storage device 1 is therefore can be either a structure that cannot be moved, such as a piece of furniture or a shelf or, preferably, a trolley or other similar device provided with wheels or the like adapted to enable easy displacement.

It can be suitably used at the inside of medical structures such as laboratories or hospitals, for handling and/or preserving medical equipment.

In detail, the storage device 1 can be used in almost all the departments present in a medical structure; for instance it is adapted to be used in the surgical departments and the anaesthesia departments, in a first aid station or in a dressing department.

It comprises a self-supporting structure **10** defining the framework of the storage device and confining an inner volume **1b** adapted to receive one or more items; a plurality of side panels **5** each of which is adapted to define at least one portion of the side surface of the inner volume 1 b; at least one diffuser apparatus **6** adapted to carry out sanitisation of at least part of the inner volume 1 b.

In particular, the self-supporting structure 10, as better explained in the following, advantageously has abutment means enabling the side panels 5 to be easily separated from said self-supporting structure and fastened thereto. This easy separation/fastening of the side panels 5 enables easy and practical access to the inner volume 1 b by the user, and therefore allows optimal washing of the volume itself.

The self-supporting structure 10 defines the base element of the storage device 1; in fact said structure, due to the sizes of its components and the selection of the materials used, has an appropriate mechanical strength, as well as bending strength and resistance to torsional stresses so that device 1 is able to bear the efforts to which it is subjected during use. In particular, the material of which the self-supporting structure 10 is made must be easily washable and characterised by an optimal hygienic coefficient. In particular, it is stainless steel and, more specifically, stainless steel AISI 304 or AISI 316.

In addition, the inner volume 1b, defined by the self-supporting structure 10, is such that it allows almost any medical device to be housed inside the storage device, such as containers for sterilisation, forms ISO, catheters, apparatus in generals, scalpels, needles and forceps.

Preferably, the self-supporting structure 10 includes at least one shelf preferably made of stainless steel AISI 304 or AISI 316 and adapted to define a rest surface 1 a; and at least one supporting column **4** adapted to bear said shelf. In particular, the self-supporting structure 10 comprises at least two shelves: an upper shelf **2** adapted to delimit said inner volume 1 b at the upper part thereof and to define a first rest surface 1 a for medical devices, and a lower shelf **3** adapted to delimit said inner volume 1 b at the lower part thereof. In particular, said three components of structure 10 are fastened to each other through releasable connecting means, such as screws, in such a manner as not to define hidden compartments where dust, bacteria or other noxious elements can lay.

If the storage device 1 is of the movable type, the self-supporting structure can have, in addition to the aforesaid components, one or more wheels **7**, preferably of the swivelling type, placed under the lower shelf 3. Alternatively, if the storage device 1 is of the fixed type, the self-supporting structure 10 has support legs or feet, not shown in the figures, adapted to keep the storage device at a raised position. In detail the legs can consist of a portion of said supporting columns 4. The storage device 1 has a plurality of supporting columns 4 having a suitably shaped tubular configuration. The supporting columns 4 preferably have bases abutting against the upper shelf 2 and the lower shelf 3. More specifically, the supporting columns 4 are substantially enclosed between shelves 2 and 3.

In particular, the sizes and the material used for making the supporting columns is adapted to ensure an appropriate mechanical strength to device 1. In particular, columns 4 are preferably made of stainless steel AISI 304 and AISI 316, or other special steel.

The tubular shape of at least one of the supporting columns 4 is adapted to define an inner chamber **4a** suitable to receive at least one diffuser apparatus 6 at least partly. In particular, it is adapted to contain said one diffuser apparatus 6 completely inside it and, more specifically, the inner chamber 4a fully holds a single apparatus 6.

The inner chamber 4a therefore has a slit **4c** formed in the portion of the supporting column 4 facing the inner volume 1b, which is adapted to enable the diffuser apparatus 6 to sanitise and/or wash, heat, blow air into, the inner volume 1 b or other similar device.

Alternatively, should the storage device 1 have one or more intermediate shelves, not shown in the figures, the inner chamber 4a can be divided into a plurality of sub-chambers each of which provided with a diffuser apparatus 6 adapted to sanitise and/or wash the portion of inner volume 1b enclosed between two shelves.

The supporting columns 4 have the outer profile suitably shaped in such a manner as to define at least one abutment means **4b** adapted to partly receive one of the side panels 5. In particular, the abutment means 4b has such a shape that it enables the side panel 5 to be fastened to column 4 at said abutment means 4b by front interlocking coupling. This interlocking coupling can be easily obtained or released by inserting or moving panel 5 into or apart from the abutment means 4b, i.e. through movement of panel 5 along a substantially rectilinear insertion direction **5a**.

In detail, said rectilinear movement along the insertion direction 5a is adapted to move the side panel 5 away from or close to the centre of gravity of the supporting structure 10. More specifically, the insertion direction 5a is substantially parallel to the perpendicular to the side surface portion defined by the side panel itself, so as to enable the operator to carry out easy movement in any direction.

In order to allow said introduction of the side panel 5 into the abutment means 4b through translation along a rectilinear insertion direction 5a, the shape of each abutment means 4b, in order to enable a front interlocking coupling, advantageously matches a corner of the side panels 5 thus ensuring perfect coupling between the supporting column 4 and panel 5.

At least one of the abutment means 4b and panel 5 is provided with a gasket of elastomer or other similar element so as to eliminate possible residual plays in said front coupling between the abutment means 4b and side panel 5. In detail, this gasket, when the side panels are fastened to the self-supporting structure 10, allows said front interlocking coupling to be sealed so as to ensure more steadiness and tightness to the coupling. In particular, both the side panel 5 and the supporting column 4 can be simultaneously provided with a gasket.

The lower shelf 3 on the opposite side relative to the inner volume 1 b, has a collection compartment **3a** adapted to receive possible refuse material, residual fluids from washing or sanitisation for example, still present in the inner volume 1b.

The lower shelf 3, for ensuring collection of the refuse material inside the collection compartment 3a thus avoiding possible stagnation, has the inner surface, i.e. the surface facing volume 1b, suitably shaped so as to form drainage grids or preferably evacuation chutes. In particular, this inner surface is wedge-shaped with a downwards facing tip. More specifically, the inner surface at the tip has one or more holes **3b** adapted to enable the refuse material to enter the collection compartment 3a.

The collection compartment 3a is advantageously provided with a tap, a plug or other similar element adapted to enable the residual refuse stored inside it to be ejected upon command. In detail, the collection compartment 3a is wedge-shaped so as to facilitate the refuse-evacuation operations preventing said refuse from remaining inside compartment 3a.

The side panels 5, in order to facilitate the above described rectilinear movement can have projections or other grip elements, such as handles **5b**, adapted to enable an operator to easily fit or remove the side panels 5 from the respective abutment means 4a.

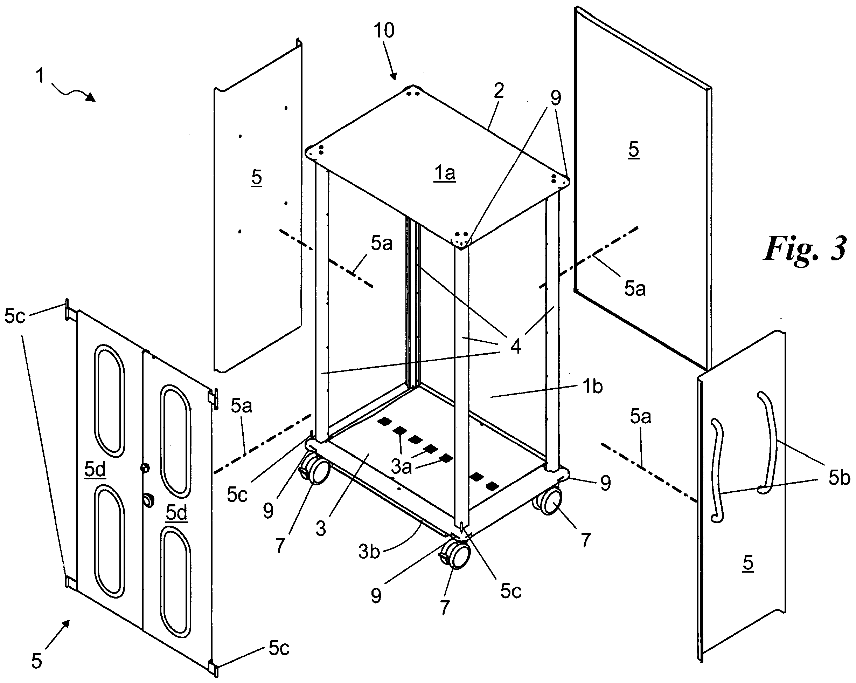

For positioning and removing objects from the inner volume 1b, one of panels 5 can be of the type adapted to be opened (Fig. 3), so that it is suitable to enable access to the inner volume 1b. In this case, this panel has connecting means **5c** such as hinges adapted to fasten it to the rest of the storage device 1, and one or more wings or doors **5d** movable relative to said means 5b for creation of said access.

In this case, the self-supporting structure 10 can be provided with one or more intermediate shelves, not shown in the figure, each of which is adapted to define a rest surface 1 a enabling a plurality of items to be disposed thereon, such as medical devices. In particular, said intermediate shelves can be positioned inside structure 10 due to the presence of suitable holes formed in columns 4 at a modular distance.

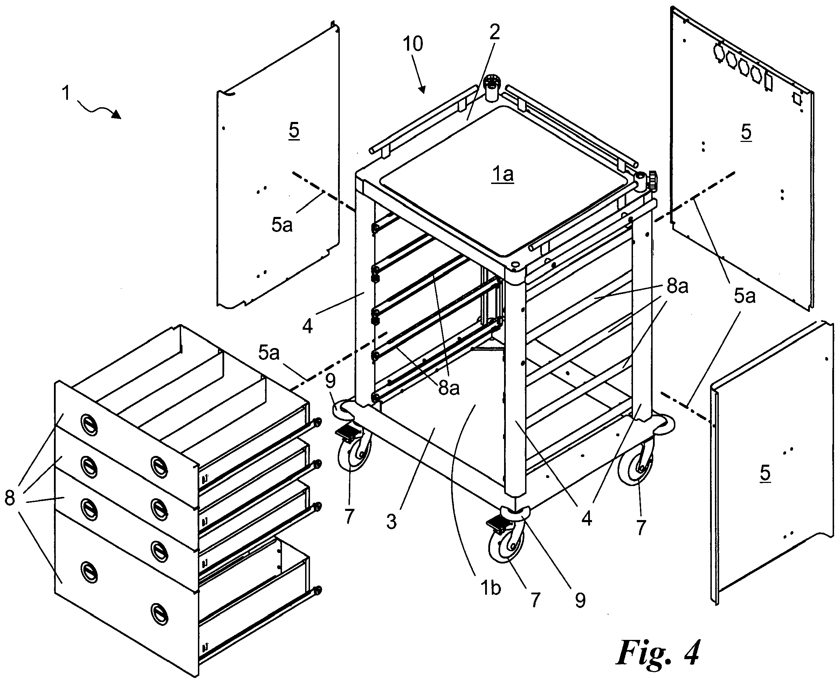

In a second preferred alternative example, arrangement of items inside the storage device 1 can be obtained through a side panel 5 provided with one or more drawers **8** (fig. 4) slidable on suitable guides **8a** secured between two supporting columns 4. In particular, guides 8a are preferably secured to column 4 in such a manner that they do not obstruct slit 4c and do not hinder sanitisation carried out by the diffuser apparatus 6.

For fastening of guides 8a or other elements, in fact, columns 4 are provided with holes for connection of accessories, disposed at a modular distance.

The diffuser apparatus 6 is adapted to sanitise and/or wash at least part of the inner volume 1 b. Preferably it sanitises the whole inner volume 1 b and the faces belonging to the different shelves 2 and 3, to the side panels and the supporting columns 4 turned towards said inner volume 1 b. The diffuser apparatus 6 is further adapted to sanitise possible items, such as medical devices, placed within volume 1 b.

The diffuser apparatus 6 is able to ensure a sure sterility or asepsis level commonly referred as SAL (Sterility Assurance Level), lower than 10⁻⁶, which means that the likelihood of finding a micro-organism is less than one out of one million.

In particular, sanitisation carried out by the diffuser apparatus 6 is of the physical type and, more specifically, of the heat-applying type. Preferably, the sanitisation process is of the type with wet heat which utilises a steam jet for bacterium elimination.

In this case the diffuser apparatus 6 can have one or more nozzles 6b disposed in the inner chambers 4a in such a manner as to sanitise and/or wash at least the inner volume 1 b and a series of ducts adapted to connect nozzles 6b for fluid passage with a feeding system in this case adapted to supply said steam, such as a sanitisation tunnel for example.

In particular, this connection is advantageously obtained through at least one connecting element **6a** such as a valve or other similar device adapted to enable said connection to be made between the feeding system and the diffuser apparatus 6. In particular, this connecting element 6a can be connected to the diffuser apparatus 6 through suitable ducts fastened to the self-supporting structure and preferably positioned in suitable housings formed in said self-supporting structure 10.

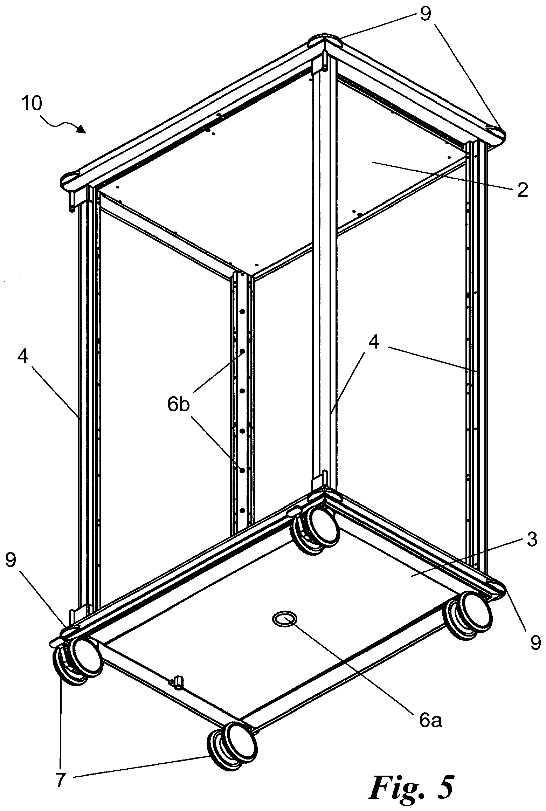

The connecting element 6a can be positioned on the outer surface of one of the shelves and, more specifically, it is placed at the outer surface of the lower shelf 3, as shown in Fig. 5. Alternatively, valve 6a is placed at one of the supporting columns 4.

Alternatively, sanitisation is carried out through a radiation system and in particular a non-ionising radiation system. This non-ionising radiation system utilises ultraviolet rays, i.e. electromagnetic radiation of wavelength almost included between 100 and 400 nm.

More specifically, said non-ionising radiation systems utilise the UV-C radiation, i.e. with a wavelength preferably substantially included between 100 and 280 nm and, in greater detail, this wavelength is almost included between 250 nm and 260 nm.

In this case, each diffuser apparatus 6 comprises one or more UV-C lamps that are suitably disposed inside the inner chambers 4a and that, through at least one connecting element 6a, are brought into communication for current passage with a feeding system that in this particular case can consist of an electric source, for example.

In this case the connecting element 6a adapted to carry out said connection for current passage is an electric outlet or other similar device. Element 6a is placed, as previously described, at one of the shelves, preferably the lower one 3, or one of columns 4.

Said electric source, not shown in the figure, can consist of a source rigidly connected to the storage device 1, a battery for example, or an outer source, such as the mains belonging to the medical structure in which the storage device 1 is used.

As an alternative to the diffuser apparatus 6 previously described, this apparatus 6 can carry out said sanitisation through hot air, liquid or heat.

Each diffuser apparatus 6 is preferably disposed in an inner chamber 4a belonging to a support column 4. In particular, if intermediate shelves are present, several sanitisation apparatus 6 can be disposed inside the inner chamber 4a so as to sanitise and/or wash each portion of the inner volume 1 b in an optimal manner.

A diffuser apparatus 6 can be placed, as an alternative to the inner chambers 4a belonging to the supporting columns 4, at one or more of the shelves present in the storage device 1. In particular, it can be placed inside the shelves, i.e. in suitable inner chambers formed in the shelves, suitably shaped and provided with openings so as to enable sanitisation of the inner volume 1 b.

Preferably, the diffuser apparatus 6 is placed in the inner chambers 4a both of the supporting columns 4 and of the shelves.

The storage device 1 can be advantageously provided with one or more elements 9 for damping possible passive energies, i.e. energies generated by possible shocks or other actions that may damage the self-supporting structure 10. In particular, the damping element 9 is adapted to absorb bounding/oscillation of wheels 7, dissipating the energy impressed to or released by the wheels themselves when they encounter ground unevenness or impact on items.

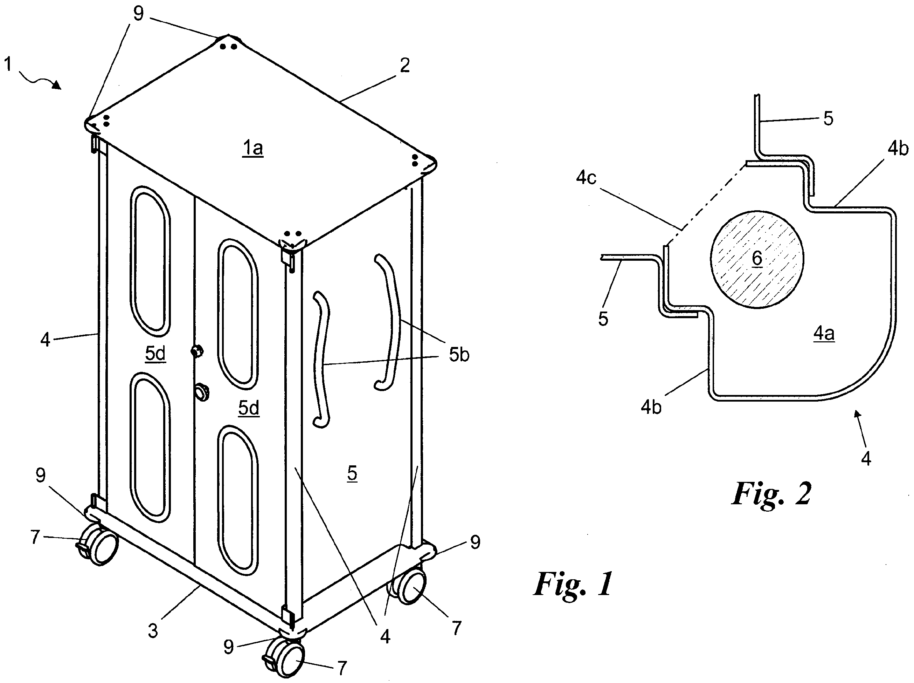

The damping element 9 is rigidly secured to the supporting structure 10 and, preferably it is placed between the supporting column 4 and at least one shelf, the lower one in particular or, alternatively, between the lower shelf 3a and wheels 7. As shown in Figs. 1, 3 and 5, the storage device 1 can contemplate the presence of a plurality of damping elements 9 disposed between the columns 4 of the lower 3 and upper 2 shelves. Alternatively, element 9 can be disposed between the lower shelf 3 and wheels 7. It consists of a disc of polymeric material or other similar element adapted to perform the aforesaid function.

Operation of the storage device described above as regards structure, is the following.

At the beginning, the operator, through fastening means, such as screws and/or welding operations, secures the supporting columns 4 to the upper shelf 2 and to the lower shelf 3 and, if any, to wheels 7 or the legs, thus obtaining the self-supporting structure 10. In particular, a damping element 9 is disposed between each supporting column 4 and the lower shelf 3.

When said self-supporting structure 10 has been finished, the side panels 5 are put in place. In particular, the operator grasps the side panels 5 by means of handles 5b and advantageously move them in the rectilinear insertion direction 5a fitting them in the respective abutment means 4b.

If inner sanitisation of the storage device 1 is wished to be carried out, it is sufficient to operate the diffuser apparatus 6 without removing the side panels 5.

The diffuser apparatus 6, due to its advantageous arrangement inside the inner chambers 4a, sanitises and/or washes the whole volume 1 b in an efficient manner. In detail, this operation allows possible items placed inside said volume to be sanitised and/or washed, in addition to the inner volume 1 b itself. Alternatively, if the whole storage device 1 is wished to be sanitised, sanitisation of same takes place by means of the thermodisinfecting machines present in the medical structure. In this case, in order to improve quality of this sanitisation operation, the side panels 5 are easily separated from the self-supporting structure 10. When separation has been completed, the self-supporting structure 10 is introduced into the thermodisinfecting machine that also sterilises the inner part of device 1 in an optimal manner.

Once sanitisation has been completed, the refuse material, due to the particular shape of the lower shelf, is automatically collected within the collection compartment 3a and subsequently ejected.

The invention comprise a new process for manufacturing a storage device 1.

According to it, a base assembling step is provided in which the self-supporting structure 10 is made. In particular, the upper shelf 2, lower shelf 3 and a plurality of supporting columns 4 are mutually fastened, thus defining the inner volume 1 b. Subsequently, a final assembling step is provided in which at least one side panel 5 is secured to the supporting columns 4, which panel defines a side surface portion of the inner volume 1b. In particular, this securing operation is achieved by front interlocking coupling of the side panels 5 in the appropriate abutment means 4b. This interlocking coupling is obtained through rectilinear movement of the side panels 5 along the above described insertion direction 5a.

The invention achieves important advantages.

In fact, the storage device 1 is of easy maintenance, washing and sanitisation.

This advantage is represented by the possibility of moving panels 5 through a simple rectilinear movement. Said advantage is obtained due to the particular abutment means 4b enabling the side panels 5 to be fastened to structure 10 through simple front interlocking coupling or, alternatively, screws or other similar connection means.

This easy replacement allows the storage device 1 to be modified quickly adapting it to the different use requirements. For instance, the side panels 5 can be replaced so as to use it for different requirements.

Since the side panels 5 can be moved, if one panel 5 is deteriorated, quick and practical replacement of this panel alone is made possible.

A fundamental improvement connected with easy removal of the side panels 5 is represented by the possibility of carrying out washing of the self-supporting structure 10 alone, and therefore of obtaining optimal sanitisation of the storage device 1 with known thermodisinfecting machines.

In addition, since the side panels 5 can be hooked and removed with ease, the structure of the storage device 1 can be modified and made suitable to each use.

Another advantage is represented by the capability of maintaining a high sanitisation level due to the impossibility of too many items, where bacteria or other dangerous micro-organisms may have their seat, being accumulated, thus giving rise to quick decay of the hygienic level of the storage device 1.

A further advantage is represented by the high sanitisation level ensured by the storage device 1. This level is the result of the particular arrangement of the diffuser apparatus 6 inside suitable inner chambers enabling the whole inner volume 1b to be sanitised with particular efficiency.

Another advantage resides in that the arrangement of apparatus 6 within said inner chambers prevents the diffuser apparatus 6 from being submitted to shock being therefore damaged.

In addition, due to the presence of the diffuser apparatus 6, sanitisation of device 1 can be carried out without resorting to thermodisinfecting machines, which will reduce the operating expenses of the device itself.

Another achieved objective is represented by the easy manipulation of the storage device 1. This advantage is also due to the reduced weight of same, thanks to the side panels made of light material and having minimum thickness, as they are not a load bearing structure exactly because there is a sturdy supporting structure 10. In addition, use of this material only for few selected components makes the storage device 1 inexpensive.

Also advantageous is the fact that since the side panels 5 are made in a manner independent of the self-supporting structure 10, they can be substantially smooth, i.e. devoid of hidden spaces or compartments. In addition, the side panels 5 thus made have no ribs or other similar elements restricting duration of same. Due to the foregoing, panels 5 can be made of any material. For instance, the can be made of wood if greater value is wished to be given to device 1, or of low cost material so as to further reduce the manufacturing costs of device 1.

In addition, since panels 5 can be removed and fastened, the compartments of the inner volume can be easily modified, for instance through insertion of drawers or intermediate shelves so that device 1 can quickly suit a plurality of uses.

A still important advantage is represented by the presence of the damping elements 9 ensuring steadiness to the storage device 1. In particular, these elements prevent the medical devices or the trolley itself possibly submitted to shocks from being damaged.

In addition, the presence of elements 9 offers a more comfortable use avoiding creaks or other irritating noise.

## Claims

1. A storage device (1) defining at least one rest surface (1a) adapted to receive a plurality of items and comprising: a self-supporting structure (10) confining an inner volume (1b) and including at least one shelf defining said at least one rest surface (1a), and a plurality of supporting columns (4) adapted to bear said shelf and comprising at least one abutment means (4b) adapted to enable at least one side panel (5) to be secured through a front interlocking coupling to said self-supporting structure (10), and adapted to enable said at least one side panel (5) to be fitted into or pulled out of said abutment means (4b) through a movement adapted to move said side panel (5) close to or away from the centre of gravity of said self-supporting structure (10) along a rectilinear fitting direction (5a); **characterised in that** at least one of said supporting columns (4) defines an inner chamber (4a), adapted to receive one diffuser apparatus (6) at least partly and adapted to carry out a sanitisation of at least part of the inner volume (1 b), and **in that** said inner chamber (4a) has a slit (4c) adapted to enable said diffuser apparatus (6) to sanitise at least said inner volume (1b).

2. A storage device (1) as claimed in claim 1, comprising at least one side panel (5), each of said panels being adapted to define at least one portion of lateral surface of said inner volume (1 b); and wherein said fitting direction (5a) is parallel to the perpendicular to said portion of lateral surface defined by said side panel (5).

3. A storage device (1) as claimed in one or more of preceding claims, wherein said inner chamber (4a) is adapted to fully receive at least one of said at least one diffuser apparatus (6).

4. A storage device (1) as claimed in one or more of preceding claims, wherein said diffuser apparatus (6) is placed at least partly inside said at least one shelf.

5. A storage device (1) as claimed in one or more of preceding claims, comprising a connecting element (6a) adapted to bring said diffuser apparatus (6) into communication with a feeding system adapted to externally generate heat, steam.

6. A storage device (1) as claimed in one or more of preceding claims, wherein said diffuser apparatus (6) comprises one or more UV-C lamps adapted to operate a sanitising operation.

7. A storage device (1) as claimed in claim 6, wherein said UV-C radiation has a wavelength substantially included between 250 nm and 260 nm.

8. A storage device (1) as claimed in claims 1-5, wherein said sanitising operation is carried out through wet heat.

9. A storage device (1) as claimed in one or more of the preceding claims, comprising a lower shelf (3) adapted to delimit said inner volume (1b) on the lower part, and wherein said lower shelf (3) has, on the opposite side relative to said inner volume (1b), a collection compartment (3a) adapted to receive refuse material present in said inner volume (1b).

10. A storage device (1) as claimed in claim 9, wherein said lower shelf (3) comprises a wedge-shape inner surface with a downward facing tip, wherein said tip comprises at least one hole (3b) adapted to enable said refuse material to enter said collection compartment (3a).

11. A storage device (1) as claimed in one or more of the preceding claims, comprising at least one damping element (9) rigidly secured to the self-supporting structure (10) and adapted to absorb passive energies.

12. A storage device (1) as claimed in claim 11, said at least one damping element (9) is placed between said supporting column (4) and said lower shelf (3).

## Patentansprüche

1. Lagerbehälter (1), der mindestens eine Ablagefläche (1 a) definiert, die eine Vielzahl an Gegenständen aufnehmen kann, und Folgendes umfasst: ein selbsttragendes Gestell (10), das ein Innenvolumen (1b) begrenzt und mindestens eine Ablage umfasst, die mindestens eine Ablagefläche (1a) definiert, sowie eine Vielzahl an Stützen (4), die diese Ablage halten; sowie mindestens einen Anschlag (4b) umfasst, um mindestens ein Seitenpaneel (5) über eine Frontalklemme an das selbsttragendes Gestell (10) anzubinden, der dazu geeignet ist, dass das mindestens eine Seitenpaneel (5) eingesteckt oder aus dem vorgenannten Anschlag (4b) herausgezogen wird, mithilfe einer Bewegung, mit der das vorgenannte Seitenpaneel (5) näher herangeschoben oder vom Schwerpunkt dieses selbsttragenden Gestells (10) eine geradlinige Einführungsrichtung (5a) entlang entfernt werden kann, **dadurch gekennzeichnet, dass** mindestens einer dieser Stützen (4) eine interne Kammer (4a) definiert, die mindestens teilweise eine Diffusorsäule (6) aufnehmen kann und zur Hygienisierung von mindestens einem Teil dieses Innenvolumen (1b) geeignet ist, sowie dadurch, dass die vorgenannte interne Kammer (4a) einen Schlitz (4c) hat, der dafür geeignet ist, dass die Diffusorsäule (6) zumindest das vorgenannte Innenvolumen (1b) hygienisch reinigen kann.

2. Lagerbehälter (1) nach Anspruch 1, der mindestens einen Seitenpaneel (5) umfasst, von denen jedes dafür geeignet ist, mindestens einen Teil der Seitenfläche des vorgenannten Innenvolumens (1b) zu definieren; und in dem die vorgenannte Einführungsrichtung (5a) parallel zur Normalen dieses Teils der Seitenfläche verläuft, die von dem vorgenannten Seitenpaneel (5) definiert wird.

3. Lagerbehälter (1) nach einem oder mehreren der vorigen Ansprüche, in dem die vorgenannte interne Kammer (4a) dazu geeignet ist, intern mindestens eine der vorgennannten Diffusorsäulen (6) aufzunehmen.

4. Lagerbehälter (1), nach einem oder mehreren der vorigen Ansprüche, in dem die vorgenannte Diffusorsäule (6) zumindest teilweise in dieser zumindest einen Ablage positioniert ist.

5. Lagerbehälter (1) nach einem oder mehreren der vorigen Ansprüche, der ein Verbindungselement (6a) umfasst, das dafür geeignet ist, die vorgenannte Diffusorsäule (6) mit einem Versorgungssystem zu verbinden, das dafür geeignet ist, außen Wärme, Dampf zu generieren.

6. Lagerbehälter (1) nach einem oder mehreren der vorigen Ansprüche, in dem die vorgenannte Diffusorsäule (6) eine oder mehrere UV-C Lampen umfasst, die zur Hygienisierung geeignet sind.

7. Lagerbehälter (1) nach einem oder mehreren der vorigen Ansprüche, in dem die vorgenannte UV-C Strahlung eine Wellenlänge hat, die im Wesentlichen zwischen 250 nm und 260 nm liegt.

8. Lagerbehälter (1) nach einem oder mehreren der Ansprüche 1-5, in dem die vorgenannte Hygienisierung über eine feuchte Wärme realisiert wird.

9. Lagerbehälter (1) nach einem oder mehreren der vorigen Ansprüche, umfassend eine untere Ablage (3), die dazu geeignet ist, das vorgenannte Innenvolumen (1b) unten zu begrenzen und in dem die untere Ablage (3) gegenüber dem erwähnten Innenvolumen (1b) eine Sammelwanne (3a) aufweist, in der Ausschussreste gesammelt werden können, die sich im Innenvolumen (1 b) befinden.

10. Lagerbehälter (1) nach Anspruch 9, in dem die untere Ablage (3) eine interne keilförmige Fläche mit der Spitze nach unten umfasst, in der diese Spitze mindestens ein Loch (3b) umfasst, das dafür geeignet ist, dass die Ausschussreste in das erwähnte Sammelfach (3a) eintreten.

11. Lagerbehälter (1) nach einem oder mehreren der vorigen Ansprüche, umfassend mindestens ein Bremselement (9), das fest an das Gestell (10) gebunden ist und mit dem passive Energie absorbiert werden kann.

12. Lagerbehälter (1) auf Anspruch 11, in dem das vorgenannte mindestens eine Bremselement (9) zwischen der Stütze (4) und der vorgenannten unteren Ablage (3) positioniert ist.

## Revendications

1. Récipient de stockage (1) définissant au moins une surface d'appui (1 a) apte à accueillir une pluralité d'objets et comprenant : une structure autoporteuse (10) délimitant un volume interne (1b) et incluant au moins une tablette définissant ladite au moins une surface d'appui (1a) et une pluralité de colonnes de support (4) aptes à soutenir ladite tablette ; et comprenant au moins une feuillure (4b) apte à permettre de solidariser au moins un panneau latéral (5) moyennant encastrement frontal dans ladite structure autoporteuse (10) et apte à permettre audit au moins un panneau latéral (5) d'être inséré ou extrait de ladite feuillure (4b) moyennant un mouvement apte à approcher ou éloigner ledit panneau latéral (5) du barycentre de ladite structure autoporteuse (10) le long d'une direction d'insertion (5a) rectiligne, **caractérisé en ce qu'**au moins une des dites colonnes de support (4) définit une chambre interne (4a) apte à accueillir au moins partiellement un appareil diffuseur (6) et apte à opérer une hygiénisation d'au moins une partie dudit volume interne (1b) et **en ce que** ladite chambre interne (4a) présente une fente (4c) apte à permettre audit appareil diffuseur d'hygiéniser au moins ledit volume interne (1 b).

2. Récipient de stockage (1) selon la revendication 1, comprenant au moins un panneau latéral (5) chacun desquels apte à définir au moins une partie de surface latérale dudit volume interne (1b) ; et dans lequel ladite direction d'insertion (5a) est parallèle à la normale à ladite partie de surface latérale définie par ledit panneau latéral (5).

3. Récipient de stockage (1) selon l'une ou plusieurs des revendications précédentes, dans lequel ladite chambre interne (4a) est apte à accueillir entièrement au moins un dudit au moins appareil diffuseur (6).

4. Récipient de stockage (1) selon l'une ou plusieurs des revendications précédentes, dans lequel ledit appareil diffuseur (6) est placé au moins partiellement à l'intérieur de ladite au moins une tablette.

5. Récipient de stockage (1) selon l'une ou plusieurs des revendications précédentes, comprenant un élément de connexion (6a) apte à mettre ledit appareil diffuseur (6) en connexion avec un système d'alimentation apte à générer extérieurement de la chaleur, de la vapeur.

6. Récipient de stockage (1) selon l'une ou plusieurs des revendications précédentes, dans lequel ledit appareil diffuseur (6) comprend une ou plusieurs lampes UV-C aptes à opérer une hygiénisation.

7. Récipient de stockage (1) selon l'une ou plusieurs des revendications précédentes, dans lequel ledit rayonnement UV-C a une longueur d'onde essentiellement comprise entre 250 nm et 260 nm.

8. Récipient de stockage (1) selon l'une ou plusieurs des revendications 1-5, dans lequel ladite hygiénisation est réalisée moyennant de la chaleur humide.

9. Récipient de stockage (1) selon l'une ou plusieurs des revendications précédentes, comprenant une tablette inférieure (3) apte à délimiter inférieurement ledit volume interne (1b) et dans lequel ladite tablette inférieure (3) présente, du côté opposé par rapport audit volume interne (1b), un compartiment de collecte (3a) apte à collecter des résidus de rebut présents dans ledit volume interne (1b).

10. Récipient de stockage (1) selon la revendication 9, dans lequel ladite tablette inférieure (3) comprend une surface interne en forme de coin avec pointe tournée vers le bas, dans lequel ladite pointe comprend au moins un trou (3b) apte à permettre auxdits résidus de rebut d'entrer à l'intérieur dudit compartiment de collecte (3a).

11. Récipient de stockage (1) selon l'une ou plusieurs des revendications précédentes, comprenant au moins un élément amortisseur (9) fixé solidairement à la structure porteuse (10) et apte à absorber des énergies passives.

12. Récipient de stockage (1) selon la revendication 11, dans lequel ledit au moins élément amortisseur (9) est placé entre ladite colonne de support (4) et ladite tablette inférieure (3).
